(19) **Europäisches Patentamt**
**European Patent Office**
**Office européen des brevets**

(11) **EP 2 808 050 A2**

(12) **EUROPEAN PATENT APPLICATION**

(43) Date of publication:
03.12.2014 Bulletin 2014/49

(51) Int Cl.:
*A61M 16/06* (2006.01)

(21) Application number: **14179466.9**

(22) Date of filing: **28.06.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2011 US 201161502961 P**
**30.06.2011 US 201161502926 P**
**16.01.2012 US 201261586876 P**
**16.01.2012 US 201261586886 P**
**16.01.2012 US 201261586932 P**
**27.03.2012 US 201261615948 P**

(62) Document number(s) of the earlier application(s) in accordance with Art. 76 EPC:
**12745908.9 / 2 726 130**

(71) Applicant: **Koninklijke Philips N.V.**
**5656 AE Eindhoven (NL)**

(72) Inventors:
• **Asvadi, Sima**
**5600 AE Eindhoven (NL)**

• **Klee, Mareike**
**5600 AE Eindhoven (NL)**
• **Willard, Nicolaas Petrus**
**5600 AE Eindhoven (NL)**
• **Van Zanten, Joyce**
**5600 AE Eindhoven (NL)**
• **Haartsen, Jacob Roger**
**5600 AE Eindhoven (NL)**
• **Hendriks, Cornelis Petrus**
**5600 AE Eindhoven (NL)**
• **Voncken, Rudolf Maria Jozef**
**5600 AE Eindhoven (NL)**

(74) Representative: **Steffen, Thomas et al**
**Philips**
**Intellectual Property & Standards**
**P.O. Box 220**
**5600 AE Eindhoven (NL)**

Remarks:
This application was filed on 01-08-2014 as a divisional application to the application mentioned under INID code 62.

(54) **User interface device providing improved load distribution functionality**

(57) A cushion member for a user interface device is provided. The cushion member is structured to provide a load distribution functionality responsive to the cushion member being donned by the user, wherein at least a portion of the cushion member has a local stiffness of less than or equal to 100 kPa/mm responsive to a stress increase on the cushion member of 1 kPa - 15 kPa.

EP 2 808 050 A2

FIG. 3

FIG. 4

**Description**

FIELD OF THE INVENTION

The present invention relates to user interface devices for transporting a gas to and/or from an airway of a user, and in particular, to a user interface device having a cushion component that provides improved load distribution functionality.

DESCRIPTION OF THE RELATED ART

A variety of respiratory masks are known that have flexible seals and cover the nose, mouth, or both of a human user. The seals, which are also commonly referred to as cushions, are intended to create a seal against the user's face. Because of the sealing effect that is created, gases can be provided at a positive pressure within the mask for delivery to the airway of the user. In addition, many respiratory masks also employ one or more cushion elements which engage other parts of the user's face/head (e.g., the forehead, cheek or chin) for facilitating connection of the respiratory mask to the user's face/head in a comfortable manner. Such cushions are typically coupled to a rigid or semi-rigid shell or frame member which provides support for the mask.

The uses for such masks range from high altitude breathing, i.e., aviation applications, to mining and fire fighting applications, to various medical diagnostic and therapeutic applications. For example, such masks are used in the delivery of continuous positive airway pressure (CPAP) or variable airway pressure (collectively often referred to as PAP), such as a bi-level pressure that varies with the user's respiratory cycle or an autotitrating pressure that varies with the monitored condition of the user. Typical pressure support therapies are provided to treat a medical disorder, such as sleep apnea syndrome, in particular, obstructive sleep apnea (OSA), or congestive heart failure. During use, such respiratory masks, also often referred to as patient interface devices, are strapped on the head of the patient in order to interface the pressure generating device (e.g., a CPAP machine) with the patient.

A patient interface device that is used to deliver pressure support therapy to a patient must have an effective seal and needs to be held on the patient's face/head securely. Many people find that wearing current patient interface devices is uncomfortable, often to such an extent that use of the device is discontinued. Many patients report skin irritation, red marks and skin breakdown caused by wearing the patient interface device, and the recovery time from such problems typically varies from minutes to hours. However, in extreme cases, longer-lasting skin damage and pressure sores can occur. These skin problems can result in low patient compliance with patient interface devices and pressure support therapy.

The root causes of the formation of red marks on the face of a person wearing a patient interface device are manifold and not yet fully understood. Common factors reported in connection with tissue breakdown are excessive mechanical skin load by pressure, shear and friction. In general, mechanical skin loading by pressure, shear and friction can lead to multiple effects, including: (i) ischemia, which is the occlusion of capillary blood vessels, (ii) reperfusion injury, wherein after unloading of the patient interface device, accumulated free radicals are released and cause inflammation and cell damage, (iii) lymphatic function impairment, wherein occlusion and damage of the lymph vessels prevents the removal of metabolic waste, leading to tissue necrosis, and (iv) mechanical deformation of tissue cells, wherein cell membranes rupture, volume changes of cells leads to initial damage, and cytoskeletal reorganization occurs. In addition, moisture accumulation on/in the skin due to coverage of the skin decreases the skin ability to resist damage.

The materials of choice in current patient interface devices, such as PAP patient interface masks, are silicone rubbers because of the biocompatibility they provide. Silicone materials, a group of materials based on various types of polysiloxanes, are typically highly stable against chemical modification and aging and, therefore, guarantee for a long shelf life and time of use. These materials, however, are intrinsically hydrophobic and have very low water permeability. As a result, moisture often accumulates at the skin/mask interface as well as in the skin, which decreases the skin strength and it's tolerance to damage. This in turn decreases comfort and increases the likelihood of skin damage and red mark formation when wearing a patient interface.

In addition to blocking of moisture, silicone materials as used in today's patient interface devices have the strong disadvantage that they have a very smooth surface, typically on the order of 0.1 microns up to several microns. These silicone materials show a high friction coefficient at the skin, which can result in high shear deformation at the skin. The high skin loading due to shear deformations thus results in an increased likelihood of red mark formation and patient discomfort. As noted above, such discomfort problems can lead to reduced therapy compliance by patients as they may wish to avoid wearing an uncomfortable mask.

SUMMARY OF THE INVENTION

Accordingly, it is an object of the present invention to provide a patient interface device that overcomes the shortcomings of conventional user interface devices. This object is achieved according to one embodiment of the present invention by providing a user interface device that provides improved load distribution, moisture transport, friction characteristics and/or cooling of the skin.

In one embodiment, a cushion member for a user interface device is provided. The cushion member is structured to provide a load distribution functionality responsive to the cushion member being donned by the user, wherein at least a portion of the cushion member has a local stiffness of less than or equal to 100 kPa/mm responsive to a stress increase on the cushion member of 1 kPa - 15 kPa

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention.

BRIEF DESCRIPTION OF THE DRAWINGS

FIGS. 1 and 2 are front and side elevational views, respectively, of a system adapted to provide a regimen of respiratory therapy to a patient according to one exemplary embodiment;

FIG. 3 is a schematic representation of a forehead cushion forming part of a respiratory mask of the system of FIGS. 1 and 2 according to one particular, non-limiting exemplary embodiment;

FIG. 4 is a schematic representation of a spacer fabric that may be used to implement a cushion of a respiratory mask of the system of FIGS. 1 and 2 according the various embodiment described herein;

FIG. 5 presents a schematic diagram of an irregularity in a forehead pad;

FIG. 6 is a graphical representation of the pressure increase caused by a forehead pad due to an irregularity for two materials having a different stiffness;

FIG. 7 is a graphical representation of the pressure increase caused by a forehead pad due to an irregularity for two materials each having a different and non-constant stiffness;

FIG. 7A provides schematic stress-displacement curves of several (typical) different materials;

FIG. 8 is a schematic representation of a forehead cushion forming part of a respiratory mask of the system of FIGS. 1 and 2 according to an alternative particular, non-limiting exemplary embodiment;

FIG. 9 is a schematic representation of forehead cushion forming part of a respiratory mask of the system of FIGS. 1 and 2 according to another alternative particular, non-limiting exemplary embodiment;

FIGS. 10, 11 and 12 are schematic diagrams of further alternative forehead cushion embodiments that may form part of a respiratory mask of the system of FIGS. 1 and 2;

FIG. 13 is a front elevational view and FIG. 14 is a cross sectional view of a sealing cushion forming part of a respiratory mask of the system of FIGS. 1 and 2 according to another particular, non-limiting exemplary embodiment;

FIG. 15 is a cross sectional view of a sealing cushion forming part of a respiratory mask of the system of FIGS. 1 and 2 according to an alternative particular, non-limiting exemplary embodiment;

FIGS. 16 and 17 are front elevational views of further alternative embodiments of a sealing cushion that may form a part of a respiratory mask of the system of FIGS. 1 and 2; and

FIG. 18 is a graph showing the moisture increase in and on the skin with standard hydrophobic silicones as well as for a number of different textiles according to the present invention applied to the skin for 20 min.

DETAILED DESCRIPTION OF EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

As used herein, the term "cushion member" shall refer to a part of or the entirety of a component of a user interface device that is structured to engage a portion of the body (e.g., the face/head) of the user of the user interface device when the user interface device is donned by the user to provide a cushioning and/or dampening effect and/or function, and shall include, without limitation, all or a part of a mask cushion (e.g., coupled to a frame such as a faceplate), a forehead cushion/pad, a cheek cushion/pad or a chin cushion/pad (including flaps that may form a part thereof).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

The present inventors have identified a number of parameters that contribute to patient interface mask discomfort. Each parameter plays a role in causing red marks on the skin. Furthermore, interaction between the parameters results in enhancing the effect of each single parameter. In what follows, these major mask discomfort parameters are listed and solutions for reducing or eliminating their effect are described. As described in greater detail herein, in engineering a mask material for reducing the likelihood of skin/tissue discomfort as a result of these parameters, special attention is paid to textile based designs as textile materials are the single most important materials for on body applications because of their unique attributes of softness, flexibility, and breathability, as well as being a familiar material to the consumer for on body applications.

The first two parameters identified by the present inventors are the moisture content and the temperature of the skin underneath the mask. Both moisture content and skin temperature increase as a result of the tight enclosed contact between the mask material and the user's skin/body. In order to minimize or overcome the unrecovered skin deformation during wear, it is therefore important that the mask design includes: (i) materials and/or mechanisms to restore natural skin moisture balance, and (ii) a means of skin cooling.

The next two parameters identified by the present inventors that contribute to patient interface mask discomfort are load distribution and friction. A certain level of load application on the skin and the tissue underneath the skin is necessary for proper functioning of a patient interface mask as it has to be securely placed on the face and head of the user. However, inhomogeneous skin loading results in the creation of pressure points that contribute to red mark formation. Moreover, relative movement of the mask material and the skin while in tight contact and under pressure also contributes to red mark formation through frictional stress. It is therefore important that the mask design provides: (i) load distribution means in order to avoid high pressure spots (as used herein, the term load distribution shall refer to the spreading of a force or stress of a load over a surface, component or material such that it is not localized at the point of application of the force or stress, and (ii) optimized friction between the mask material and the skin.

Described herein are a number of embodiments of solutions that are specifically designed to minimize or eliminate one or multiple causes of skin irritation and discomfort as described above during wearing of a patient interface mask.

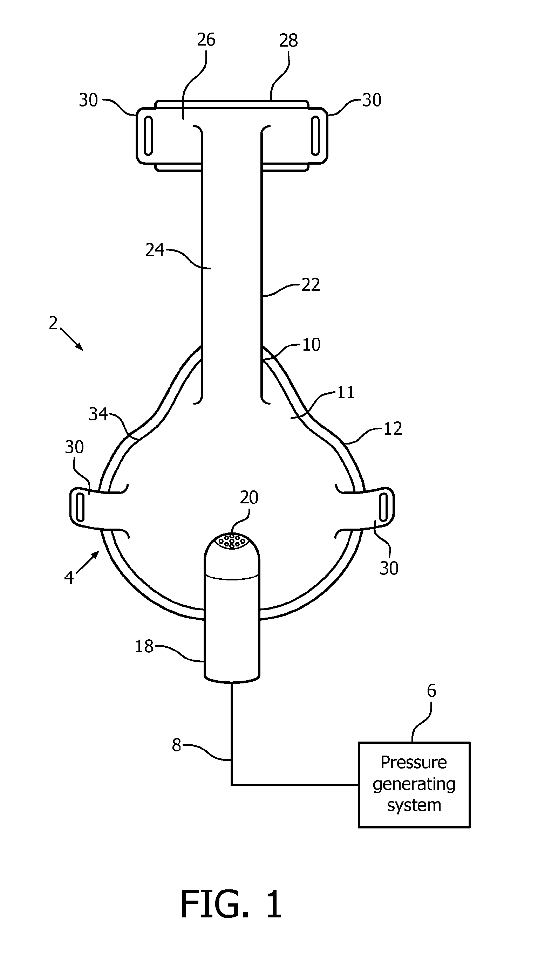

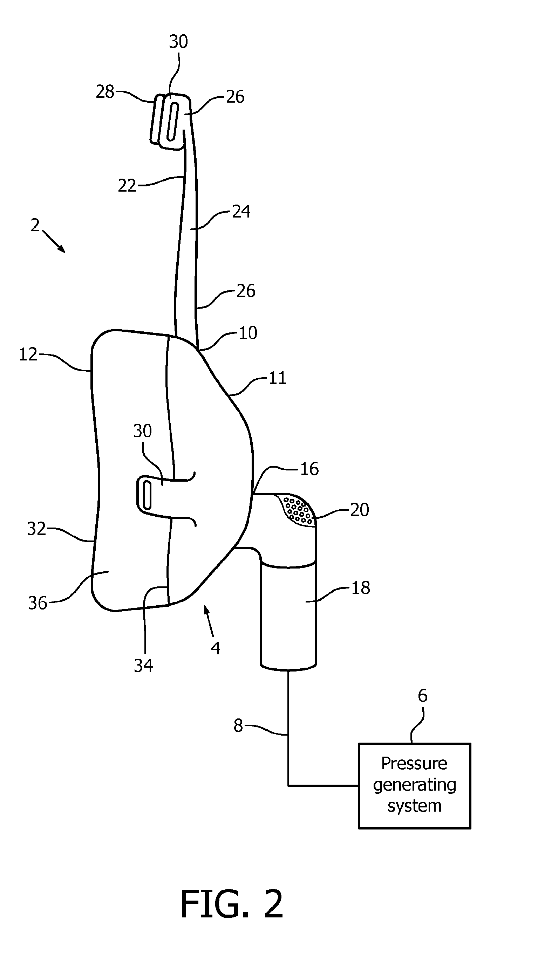

FIGS. 1 and 2 are front and side elevational views, respectively, of a system 2 adapted to provide a regimen of respiratory therapy to a patient according to one exemplary embodiment of the present invention. As seen in FIGS. 1 and 2, system 2 includes a respiratory mask 4, also referred to as a patient interface device, according to one exemplary embodiment that is shown schematically attached to a pressure generating system 6 via a user circuit 8, as is conventionally known in the art. Pressure generating system 6 is any device capable of generating a flow of breathing gas or providing gas at an elevated pressure. Examples of such pressure generating systems include ventilators, constant pressure support devices (such as a continuous positive airway pressure device, or CPAP device) in which the pressure provided to the user is constant over the user's respiratory cycle, and variable pressure devices (e.g., BiPAP®, Bi-Flex®, or C-Flex™ devices manufactured and distributed by Philips Respironics of Murrysville, Pennsylvania) in which the pressure provided to the user varies with the user's respiratory cycle, and auto-titration pressure support devices.

As seen in FIGS. 1 and 2, respiratory mask 4 includes a shell or frame assembly 10 and a cushion 12 attached to frame assembly 10. Frame assembly 10 includes a faceplate portion 11. User circuit 8 is coupled to a port 16 defined in faceplate portion 11, and, in the illustrated embodiment, includes an elbow connector 18 for that purpose. In the exemplary embodiment, user circuit 8 is connected to frame assembly 10 so as to pivot or rotate relative to frame assembly 10, and may or may not be detachable therefrom. In short, any suitable coupling technique for joining user circuit 8 to frame assembly 10 is contemplated by the present invention.

In the illustrated exemplary embodiment, an exhaust vent 20 is provided in elbow connector 18 for exhausting a flow of gas from mask 4 to ambient atmosphere. Such exhaust vents are conventionally used in pressure support systems that use a single-limb, i.e., a single conduit, to communicate a flow of gas to an airway of a user. Thus, the present invention contemplates that exhaust vent 20 can be any suitable exhaust vent, and can be located not only on elbow connector 10, but alternatively on another part of respiratory mask 4, such as on frame assembly 10.

Respiratory mask 4 can have any one of a number of different configurations, shapes, and sizes. In the illustrated, exemplary embodiment, respiratory mask 4 is a nasal/oral mask structured to cover the nose and mouth of the patient. However, other types of respiratory masks, such as, without limitation, a nasal mask, a nasal cushion or a full face mask, which facilitate the delivery of the flow of breathing gas to the airway of a patient, may be substituted for respiratory mask 4 while remaining within the scope of the present invention.

Frame assembly 10, in the exemplary embodiment, is formed from a rigid or semi-rigid material, such as a polycarbonate or an injection molded thermoplastic. In addition, as seen in FIGS. 1 and 2, frame assembly 10 includes a forehead support assembly 22. The forehead support assembly 22 is generally T-shaped and includes a support arm 24 which extends form faceplate portion 11 and which is coupled to a forehead support bracket 26. Forehead support bracket 26 includes a forehead cushion 28 disposed on the user contacting side to engage the forehead of the user. Forehead cushion 28, in various embodiments, is described in greater detail herein and is designed to minimize or eliminate one or multiple causes of skin irritation and discomfort during wearing of a respiratory mask 4.

In the illustrated, exemplary embodiment, a headgear (not shown) attaches to respiratory mask 4 via headgear connectors 30. Headgear connectors 30 attach to straps (not shown) of the headgear, for example by inserting the straps into slots provided in headgear connectors 30. In the illustrated embodiment, headgear connectors 30 are attached to each side of forehead support bracket 26 and to each side of the lower portion of frame assembly 10.

Cushion 12, also referred to as a seal or sealing member, is described in greater detail herein and, like forehead cushion 28, is, in various embodiments, designed to minimize or eliminate one or multiple causes of skin irritation and discomfort during wearing of a respiratory mask 4. As seen in FIG. 2, cushion 12 includes a first end portion 32 structured to sealingly engage the patient's face, a second end portion 34 opposite first end portion 32 that couples to the rear of frame assembly 10, and a sidewall portion 36 extending between first end portion 32 and second end portion 34 such that cushion 12 defines an inner chamber. In the illustrated, non-limiting embodiment, faceplate portion 11 and cushion 12 are generally triangular-shaped and thus each includes an apex region, a bottom region opposite the apex region, and first and second opposite side regions. When coupled to frame assembly 10, the inner chamber of cushion 12 receives the nose and mouth of the user when respiratory mask 4 is donned by the user so that the user's airway is in fluid communication with the chamber.

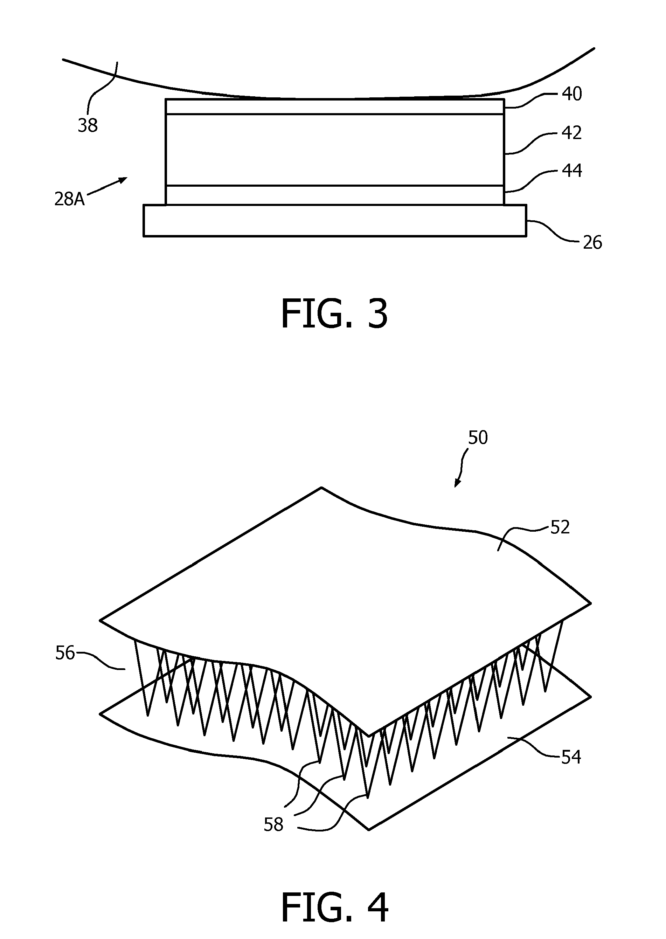

FIG. 3 is a schematic representation of forehead cushion 28A according to one particular, non-limiting exemplary embodiment. In FIG. 3, forehead cushion 28A is shown as engaging the forehead 38 of the user/patient. Because there is no airflow in forehead cushion 28A (in contrast to cushion 12), there is no need to ensure a near air tight seal between forehead pad 28A and the skin.

As seen in FIG. 3, forehead cushion 28A according to the present embodiment has a three layer configuration comprising a first layer 40 structured to engage the skin, a second, intermediate layer 42 structured to provide a load distribution and/or an air or water transport functionality, and a third layer 44 structured to engage and facilitate attachment to forehead support bracket 26. Each of the layers 40, 42, 44 of the present embodiment is described in greater detail below.

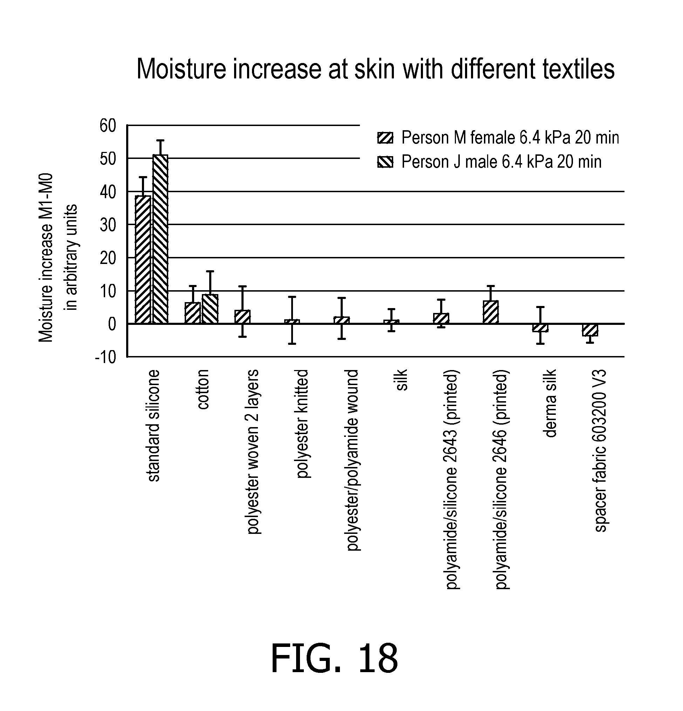

In one particular implementation of the various embodiments described in detail herein, first layer 40 and second layer 42 are structured such that the moisture increase in and on the skin of the user of respiratory mask 4 will be low, for example in the order of 0.0 - 30.0 in arbitrary units (or alternatively, 0.0 - 20.0 in arbitrary units) after 20 min usage of the material compared to a moisture increase in the order of a minimum of 40-50 in arbitrary units after 20 min usage of a standard hydrophobic silicone material applied in today's patient interfaces after 20 min of usage of the respiratory mask. The moisture increase used here is based on the measurement of the skin hydration or skin moisture with a corneometer after a patient device is applied to the skin (e.g., the forehead) for 20 min and subtracted from the skin hydration or skin moisture values measured with a corneometer before a patient device is applied to the skin. As is known in the art, a corneometer measures on capacitance of a dielectric medium and determines skin hydration based from any change in the dielectric constant. Some corneometers only report a corneo value for their output, basically an "arbitrary unit." The moisture increase in and on the skin with standard hydrophobic silicones as well as for a number of different textiles according to the present invention applied to the skin for 20 min is shown graphically in FIG. 18.

As noted above, first layer 40 is structured to engage the skin (i.e., forehead 38) of the wearer and is made of a textile material. As used herein, the term "textile" shall mean a material consisting of a network of interlaced or otherwise entangled natural or artificial fibers made by, for example and without limitation, weaving, knitting, spreading, crocheting, or bonding (e.g., by chemical, mechanical, heat or solvent treatment) the fibers to form the network, and may include, for example, and without limitation, woven and nonwoven fabric materials.

In one particular exemplary embodiment (referred to as "Embodiment 40A"), first layer 40 is made of a fabric material made of staple or filament (mono or multi filament) yarns by weaving, knitting or braiding. The fiber content of such yarn provides an inherently moisture absorbing (hydrophilic) material, and may include fibers such as, without limitation, cotton, or other natural cellulose fibers such as linen and bamboo plus other regenerated cellulosic fibers such as viscose rayon, animal fibers such as wool, or silk. In the literature, hydrophilicity of fibers is reported as either a % moisture content or a regain value. The level of each of these measures varies with the environmental conditions of relative humidity (RH) and temperature. For example cotton fiber can typically have a % moisture regain value of 8% at 65% RH and 21°C temperature. This value can go up to 20%-25%, as a function of an increase in environmental RH value (to 95% RH for example), before the fabric begins to feel damp. The moisture content of viscose rayon under the same conditions (65% RH, 21°C) is 11%. Wool and silk fibers can have % moisture regain values of 11-13% under these conditions and their % moisture regain value can reach a minimum of 30% before they feel damp. As used herein, "% moisture content" and "% moisture regain value" shall mean and shall be determined based on the following:

$$\% \text{ moisture content} = ((\text{mass of wet fiber} - \text{mass of dry fiber})/\text{mass of wet fiber})) \times 100;$$

$$\% \text{ moisture regain value} = ((\text{mass of wet fiber} - \text{mass of dry fiber}) /\text{mass of dry fiber})) \times 100$$

In one particular, non-limiting implementation, the textile material in this Embodiment 40A will have a % moisture regain value of 7.0%-11.0% at 65% RH and 21°C temperature. In another particular, non-limiting implementation, the textile material in this Embodiment 40A will have a % moisture regain value of 11%-18% at 65% RH and 21°C temperature.

Alternatively, the textile material in this Embodiment 40A can be a modified synthetic fiber that has been altered chemically or by means of surface modification methods (such as plasma treatment) so as to render it at least partially hydrophilic. For example, polyester is a hydrophobic polymer for which a % moisture regain value of 0.4% has been reported at 65% RH and 20°C temperature. Through modifications such as plasma treatment, co-polymerization and antistatic finishing moisture absorption of, polyester can be enhanced to a point where the % moisture regain value is 2-4%. Thus, as used herein in connection with this embodiment, "at least partially hydrophilic" shall mean a % moisture regain value of 2-4% or higher.

As a further alternative, the fabric material in this Embodiment 40A can be a non-woven fabric with "medium openness" that is produced by any known or hereafter developed nonwoven manufacturing method and that is based on a hydrophilic fiber (e.g. viscose) or has a mixed component fiber content such as polyester/cotton wherein at least one fiber is a hydrophilic fiber. As used herein, the term "openness" (sometimes referred to as an "openness factor") shall mean the portion or percentage of the textile material that is open space (as opposed to a fiber). For example, a textile with an openness of 10% means that the textile is 10% open space (i.e., 10% voids). In one particular implementation of this Embodiment 40A, the nonwoven fabric material with medium openness may have an openness of 75- 90%.

If first layer 40 hydrophilic, as in embodiment 40A as just described, its main function would be to prevent moisture accumulation in and on the skin by absorbing and retaining it within its fibers and thus prevent increased skin hydration.

In another particular exemplary embodiment (referred to as "Embodiment 40B"), first layer 40 is made of a textile material made of inherently hydrophobic fiber/yarn, such as polyester, polyamide, or spandex, or a partially hydrophobic fiber, such as nylon. In this Embodiment 40B, the textile material exhibits a particular "high openness" in order to provide moisture absorption and/or moisture transport properties. In the exemplary embodiment, the textile material has a high openness of 90-99% (thus making 10-75% low openness).

In another particular exemplary embodiment (referred to as "Embodiment 40C"), first layer 40 according to either Embodiment 40A or 40 B is also made of a textile material that has a certain level of fabric porosity to complement the fiber/yarn properties and allow a certain minimum level of gas (air) and water passage through first layer 40. Fabric porosity in each case is governed by fabric weight (e.g., g/m$^2$), which in turn is determined by number of yarns per unit measure (e.g., cm), also known as fabric or yarn density. Assuming that the body generates 11-15 g/m$^2$.hr of skin moisture, a cotton based fabric needs to have minimum weight of 55-75 g/m$^2$ if the fabric needs to absorb all the skin moisture (no moisture transport) and stay dry (for one hour use). A silk or wool fabric with minimum weight of 33-45 g/m$^2$ will have the same effect. Thus, in one exemplary implementation of this Embodiment 40C, the textile material has a fabric weight of greater than or equal to 30 g/m$^2$ or a fabric density of greater than or equal to 50 g/m$^2$ in order to provide the minimum level of gas (air) and water passage. In addition, as specified above in connection with Embodiments 40A and 40B, the textile material will also have either a "medium openness" (40A; 75-90%) or a "high openness" (40B; 90-99%) (e.g., as determined by number and spacing of the weft and warp yarns in a woven fabric or the number and spacing of the coarse and wale in a knitted fabric) in order to provide for sufficient moisture transport from the skin surface. This combination of parameters will facilitate moisture absorption on the one hand and moisture transport from the skin surface on the other hand.

Assuming density range of 1.1-1.5 (most synthetic fibers such as PE, Nylon, Acrylic have density around 1.1 g/cm$^3$, silk and wool 1.3 and cotton 1.5 g/cm$^3$) and fabric thickness in the range of 0.1- 20 mm, if fabrics with weight in (g/m$^2$), for example (minimum) fabric weight of 30-50 g/m$^2$ (given previously for hydrophilic materials), are used, openness/porosity values may be calculated as the ratio of % void to % (solid) material. For example a wool fabric with 35 g/m$^2$ weight and 1.0 mm thickness will have 2.7% material and 97.3% void (thus around 97% openness or porosity). If the fabric is a cotton fabric with 300 g/m$^2$ weight and thickness 2.0 mm, the openness value will be 90%. This has been calculated in the following manner (using wool as the example). A fabric that is 1.0m x 1.0m and that has a 1.0mm thickness will have a fabric volume of: 100 cm x 100 cm x 0.1 cm = 10$^3$ cm$^3$. The fabric weight if has zero openness, as in a wool slab, would be: weight = volume x density (here wool) which gives weight with zero openness = 10$^3$ cm3 x 1.3 g/ cm$^3$ = 1.3 x 10$^3$ g = 1300 g. So, if a wool fabric (with a certain openness, i.e., some void content) has a weight of 35 g/m2, then the void ration is solid material % = weight fabric with void / weight fabric with zero void x 100. Thus, solid material % = (35/1300) x 100 = 2.7 %, which means the rest of the material is void or openness (97.3%). This calculation was repeated for the other example (cotton). The same holds for hydrophobic materials although there is no minimum weight given as they absorb very limited amount of moisture.

As noted above, if the fabric material that is used to implement this Embodiment 40C is inherently hydrophobic (i.e., Embodiment 40B), it should have a high openness in order to facilitate moisture transport by, for example, wicking to keep the skin dry. As also noted elsewhere herein, in one particular implementation, the "high openness" is an openness of 90-99%. For example, the present inventors have tested a number of fabrics including two polyester fabrics. Both fabrics resulted in almost zero increase in moisture accumulation on and in the skin as against silicone that resulted in 40-50 (in arbitrary units) increase in moisture accumulation compared with textiles. The moisture increase used here is determined by measuring the skin hydration or skin moisture with the corneometer after a textile or hydrophobic silicone is applied to the skin for 20 min and subtracted from the skin hydration or skin moisture values measured with a corneometer before the textile or hydrophobic silicone is applied to the skin.

In another particular exemplary embodiment (referred to as "Embodiment 40D"), first layer 40 is made of a textile material that has a textile surface having optimum smoothness in such a way that minimizes skin-fabric friction. In one exemplary embodiment, the optimum smoothness is provided by a textile material that has a skin contacting surface having a coefficient of static friction value of less than or equal to 2.0 between the skin contacting surface and the skin, for example as measured with textile materials on the facial skin (e.g. the forehead) with a reciprocating skin friction tester, using a measurement principle as for instance described in M. Kwiatkowska, S.E. Franklin, C.P. Hendriks, K. Kwiatkowski, Wear 267 (2009) 1264-1273. In another exemplary embodiment, the optimum smoothness is provided by a textile material that has a skin contacting surface having a coefficient of static friction value of less than or equal to 1.0 (or 0.5) between the skin contacting surface and the skin, for example as measured as just described. The optimized material has the desired yarn properties (surface properties, yarn twist, surface treatments) in order to achieve the level of smoothness just described. In the exemplary embodiment, the fabric surface is achieved by providing a surface treatment to the fabric material. The surface treatment can have a chemical nature (e.g. through coatings) or a mechanical nature (through brushing). Alternatively, the fabric material can have a piled or looped surface structure attached to a mesh backing material of high openness (90-99%) to provide the optimum fabric surface and therefore ensure soft touch.

Thus, in the exemplary embodiment, the first layers 40A-40D just described are structured such that they are capable of providing moisture uptake from human skin at a rate of at least 11 g/m$^2$.hr in order to prevent undesirable moisture accumulation and thus undesirable skin hydration at areas of the skin that are covered by forehead cushion 28A (as will be appreciated, such moisture accumulation will result from such covered areas becoming warmer and causing the user to sweat (as opposed to, for example, moisture that might accumulate due to air exhaled by the user)). In particular alternative embodiments, they are capable of providing moisture uptake from human skin at 11-15 g/m$^2$.hr, 30-40 g/m$^2$.hr, or greater than or equal to 40 g/m$^2$.hr. In addition, according to another aspect, and based on this moisture uptake value, forehead cushion 28 is able to store/retain a certain amount minimum amount of moisture. In the exemplary embodiment, forehead cushion 28 is able to store/retain at least 88 g/m$^2$ of moisture (water), e.g., 88-320 g/m$^2$ of moisture (water) or higher, which corresponds to eight hours of use at the moisture uptake rates specified above. In this embodiment, the moisture may be stored by first layer 40, second layer 42 (if present), or some combination of first layer 40 and second layer 42 (if present).

In another particular exemplary embodiment (referred to as "Embodiment 40E"), any of the embodiments 40A-40D just described may further have a surface treatment (such as soil release (FC based finishes) or oil repellent and/or any known or hereafter developed textile finishing treatment that would make the surface self-cleaning or easy to clean) applied thereto to ensure hygiene and/or facilitate cleaning of first layer 40 (and, possibly also second layer 42).

In still another particular exemplary embodiment (referred to as "Embodiment 40F"), any of the embodiments 40A-40E just described may further include a self cleaning surface coating or an antibacterial coating such as sliver ion coating or other surface treatments that would render first layer 40 (and, possibly also second layer 42) resistant to bacterial growth in order to prevent bacteria based skin irritation to the patient.

As noted elsewhere herein, second layer 42 is provided immediately adjacent to first layer 40 and is structured to provide a load distribution and/or an air or water transport functionality. Since second layer 42 is attached underneath first layer 40, it does not directly contact the skin (i.e., forehead 38) of the wearer. In the exemplary embodiment, second layer 42 is made of a spacer fabric (as described herein), a nonwoven textile, a foam material or a gel material, such as silicone gel or polyurethane gel, that mainly serves to distribute load and/or transport air or water to keep the skin optimally dry and cool on the one hand and avoid pressure spots on the other. As used herein, the term "foam" shall mean a material that is formed by trapping gas in a liquid or solid in a divided form, i.e. by forming gas regions inside liquid or solid regions, leading to different kinds of dispersed media. In addition, as used herein, the term "gel" shall mean is a solid, jelly-like material defined as a substantially dilute cross-linked system which exhibits no flow when in the steady-state.

In one particular exemplary embodiment (referred to as "Embodiment 42A"), second layer 42 is made of a spacer fabric. The term "spacer fabric" as used herein shall, referring to FIG. 4, mean a three dimensional fabric structure 50 which consists of two ground fabrics (upper fabric layer 52 and lower fabric layer 54 in FIG. 4) simultaneously woven or knitted and otherwise connected together by an inner layer 56 of fibers (e.g., filament pile yarns) 58 to form a structure in three directions in a single process. Upper fabric layer 52 and lower fabric layer 54 can have different structures in terms of their surface texture, pattern, color, density, etc. The interconnect fibers or yarns 58 hold the structure in the z direction, which determines the height of the spacer fabric. The inner layer 56 comprising the fibers 58 can take a variety of shapes including tubes, pleats or other engineered forms. The spacer fabric can therefore be engineered for specific characteristics such as direction of stretch, absorbency, liquid movement, and pressure distribution. The materials used to make the spacer fabric in Embodiment 42A may be, for example, and without limitation, a polyamide material, a polyester material, a polyurethane material, or a spandex material. In one particular implementation of this Embodiment 42A, the thickness of second layer 42 is 0.1-20.0 mm (e.g., in various alternative implementations, the thickness of second layer 42 may be 0.1-1 mm, 1-6 mm, 6-15 mm or 15-20 mm).

In another particular exemplary embodiment (referred to as "Embodiment 42B"), second layer 42 is made of a foam (e.g., without limitation, a silicone or polyurethane foam) or a gel (e.g., without limitation, silicone gel or polyurethane gel) spacer material. In this Embodiment 42B, like Embodiment 42A, the spacer foam or gel material has certain specified thickness, compression resistance and elasticity parameters in order to distribute the pressure required to keep the structure on the face via head straps or other means of attachment. In one particular implementation of this Embodiment 42B, the thickness is 0.1-20.0 mm (e.g., in various alternative implementations, the thickness of second layer 42 may be 0.1-1 mm, 1-6 mm, 6-15 mm or 15-20 mm). In addition, because foams and gels typically lack the openness of a textile material (Embodiment 42A), in another exemplary implementation, second layer 42 comprising the spacer foam or gel material of Embodiment 42B is provided with a number of openings that extend therethrough to ensure moisture transport and some level of air flow for skin cooling. The number, size and spacing of such openings will depend on the particular material used and the desired level of moisture transport and/or air flow.

Furthermore, in order to prevent pressure spots and fulfill the requirement of load distribution, second layer 42 in one exemplary embodiment needs to have certain characteristics under compression load (i.e., compression resistance). This can be presented in the compressibility value that is defined as the inverse of the stiffness ($dx/d\sigma$), where stiffness is defined as the ratio of stress (load/area) and displacement (mm) in a compression deformation. Since this compressibility is a property of second layer 42, it can be used, in combination with thickness and other material properties, to engineer second layer 42 (e.g., as a spacer fabric, as a nonwoven fabric or felt or as a foam or gel layer).

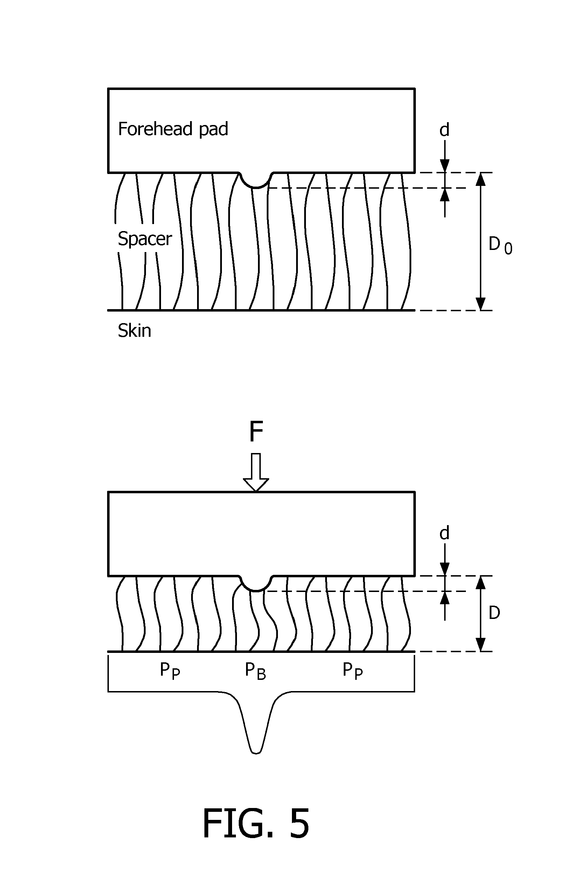

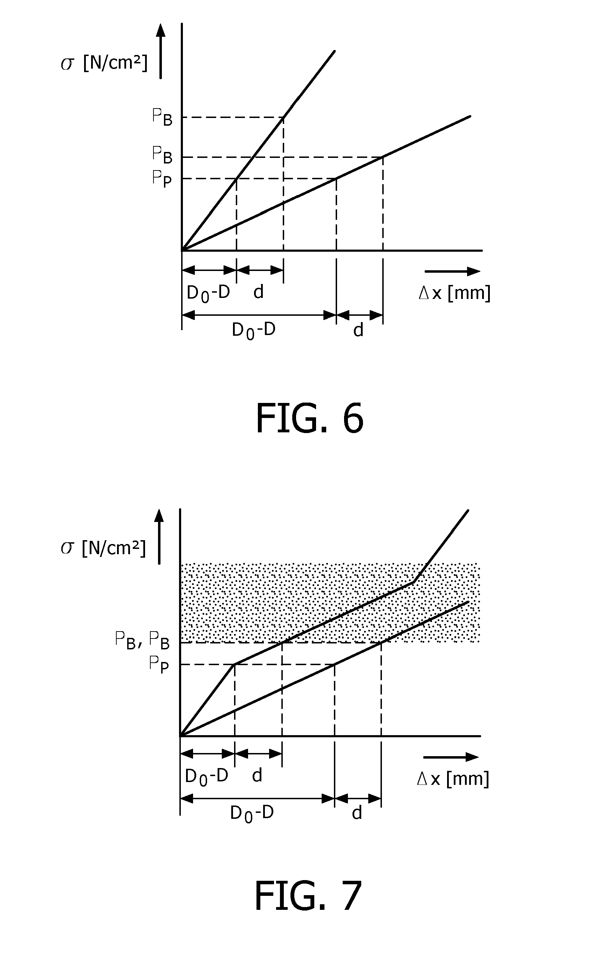

In particular, FIG. 5 presents a schematic diagram of an irregularity in a forehead pad. This asperity can be caused by the fact that the (original) shapes of the forehead pad and the skin do not match but also by a deformation of the forehead and/or the skin during loading such that the deformed shapes do not match anymore. Both situations will lead to pressure peaks at the points of skin contact. When pressing the forehead pad to the skin as shown in FIG. 5, a stress concentration will occur under the irregularity with height d. In FIG. 6, it is shown in a graphical way that this leads to a stress increase which is higher for a stiffer spacer. Therefore, it is important for avoiding pressure spots that the stiffness of second layer 42 is smaller than a certain predetermined value.

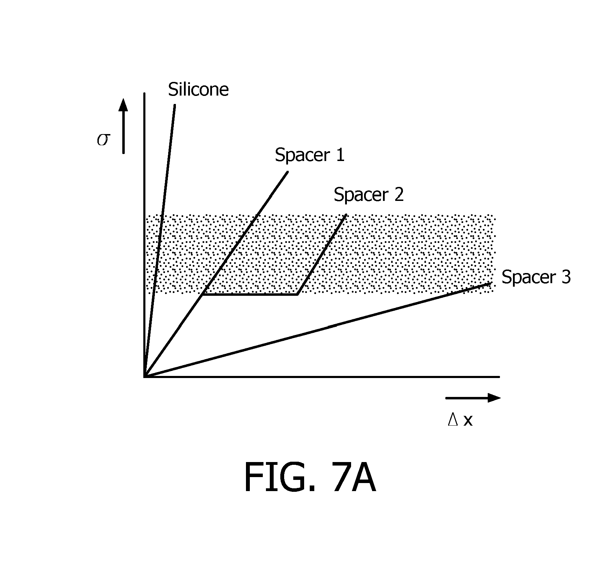

In a further embodiment of the present invention, materials are considered that show a non-constant stiffness during compression as illustrated by the top line in FIG. 7. Here, the grey shaded area depicts the threshold level for redmark formation to occur. It is important that the bending point of the curve is below the redness threshold level. As described in more detail below in connection with FIG. 7A, ideally, the local stiffness is zero at a force level just below the redness threshold level as in this case there is no or very little local stress increase at an irregularity. This property is used to combine a sufficiently low (local/tangential) stiffness at the operating compressive stress with a small total compression. This enables a spacer second layer 42 with a small layer thickness, which might be requested to apply the textile in areas of the patient interface where a thick layer cannot be applied. Thus, according to one particular, non-limiting exemplary embodiment, an upper bound for the (local/tangential) stiffness of second layer 42 to be able to reduce pressure peaks can be found when taking a maximum stress increase of $\leq 10$ kPa ($\approx$ redness threshold level) at an irregularity height d > 0.1 mm. Hence, an allowable maximum local stiffness in this exemplary embodiment is 100 kPa/mm, which corresponds to a minimum local compressibility of 0.01 mm/kPa.

Furthermore, TABLE 1 provided below sets forth a number of alternative exemplary embodiments for local stiffness of second layer 42 responsive to certain stress increases on the second layer 42.

| TABLE 1 | |
|---|---|
| **Stress Increase on Second Layer** | **Local Stiffness of Second Layer** |
| 1 kPa -15 kPa | (i) 100 kPa/mm or less, or (ii) 20 kPa/mm or less, or (iii) 7 kPa/mm or less, or (iv) 1 kPa/mm or less, or (v) 0.1 kPa/mm or less |
| 5 kPa - 15 kPa | (i) 100 kPa/mm or less, or (ii) 20 kPa/mm or less, or (iii) 7 kPa/mm or less, or (iv) 1 kPa/mm or less, or (v) 0.1 kPa/mm or less |
| 1 kPa or more | (i) 100 kPa/mm or less, or (ii) 20 kPa/mm or less, or (iii) 7 kPa/mm or less, or (iv) 1 kPa/mm or less, or (v) 0.1 kPa/mm or less |
| 5 kPa | (i) 100 kPa/mm or less, or (ii) 20 kPa/mm or less, or (iii) 7 kPa/mm or less, or (iv) 1 kPa/mm or less, or (v) 0.1 kPa/mm or less |
| 7 kPa | (i) 100 kPa/mm or less, or (ii) 20 kPa/mm or less, or (iii) 7 kPa/mm or less, or (iv) 1 kPa/mm or less, or (v) 0.1 kPa/mm or less |
| 10 kPa | (i) 100 kPa/mm or less, or (ii) 20 kPa/mm or less, or (iii) 7 kPa/mm or less, or (iv) 1 kPa/mm or less, or (v) 0.1 kPa/mm or less |
| 12 kPa | (i) 100 kPa/mm or less, or (ii) 20 kPa/mm or less, or (iii) 7 kPa/mm or less, or (iv) 1 kPa/mm or less, or (v) 0.1 kPa/mm or less |
| 15 kPa | (i) 100 kPa/mm or less, or (ii) 20 kPa/mm or less, or (iii) 7 kPa/mm or less, or (iv) 1 kPa/mm or less, or (v) 0.1 kPa/mm or less |
| 15 kPa or higher | (i) 100 kPa/mm or less, or (ii) 20 kPa/mm or less, or (iii) 7 kPa/mm or less, or (iv) 1 kPa/mm or less, or (v) 0.1 kPa/mm or less |

Furthermore, FIG. 7A provides schematic stress-displacement curves of several (typical) different materials. Those materials are identified in FIG. 7A and include a stiff material (e.g., silicone Shore 40) and three other materials, e.g., various spacer fabrics, labeled Spacer Fabric 1, Spacer Fabric 2, and Spacer Fabric 3 . As seen in FIG. 7A, the displacement curve of Spacer Fabric 2 has a plateau in the stress region just below the region where red mark formation typically begins, shown as the grey band (e.g., from about 0.6 - 1.0 $N.cm^{-2}$). In that plateau region, the slope of the curve is very small, ideally zero, which means that in that region the spacer fabric has a low stiffness and is most compressible. This low local stiffness (e.g., in the ranges specified elsewhere herein in TABLE 1) below the typical red mark formation stress range greatly helps to avoid press spots and red mark formation since the spacer fabric will able to deform rapidly. In addition, as seen in FIG. 7A, the slope of the displacement curve of Spacer Fabric 2 increases greatly at a certain level of compression. This means that at this level of compression, the stiffness of Spacer Fabric 2 increases greatly and the compressibility decreases greatly. This is beneficial when a spacer fabric is used in the second layer 42 because it will prevent (limit) the second layer 42 from compressing too much at higher stress levels. If the second layer 42 were able to compress too much (become too thin) at higher stress levels, it may undesirably decrease the ability of the second layer 42 to allow air and/or water to pass through it, and thus limit the air/moisture transport capabilities of the second layer. It should also be noted that, as seen with Spacer Fabric 2, the slope of the displacement curve may be large before the plateau region.

Thus, according to one particular exemplary embodiment, second layer 42 is chosen/designed such that it exhibits a stress-displacement curve wherein the curve has a plateau region having a significantly decreased slope (e.g., ideally decreased to zero and in one particular implementation to a slope of substantially zero, which in one implementation shall mean slopes from 0.0 to 0.03) in the stress region just below the region where red mark formation typically begins (0.6 to 1.0 $N.cm^{-2}$). Second layer 42 may have this characteristic when it is implemented as a spacer fabric as described elsewhere herein, or, alternatively, when it is implemented as a gel, foam, or non-woven fabric layer as described elsewhere herein.

As noted elsewhere herein, third layer 44 is provided immediately adjacent to second layer 42 and is provided either as means of enclosure and/or support for the first layer 40 and second layer 42 or to function as an interface between first layer 40 and second layer 42 and forehead support bracket 26 (i.e., as an integration facilitator). In the exemplary embodiment, third layer 44 is a polymer layer (based on, e.g., without limitation, silicone or TPU). Third layer 44 can be printed on second layer 42 (e.g. by transfer printing, screen printing, inkjet printing or any other method of printing that is used to print polymer based finishes on textile based materials). Alternatively, third layer 44 can be coated or laminated on second layer 42 by any of the coating methods that are known or hereafter developed in textile finishing industry for textile coating.

It should be noted that a forehead cushion 28A can be made using any of the particular embodiments of layers 40, 42, 44 described herein in any combination thereof that is deemed suitable for the application in question. Thus, any of Embodiments 40A-40 F can be used with any of Embodiments 42A, 42B and/or any of the embodiments of third layer 44 described herein.

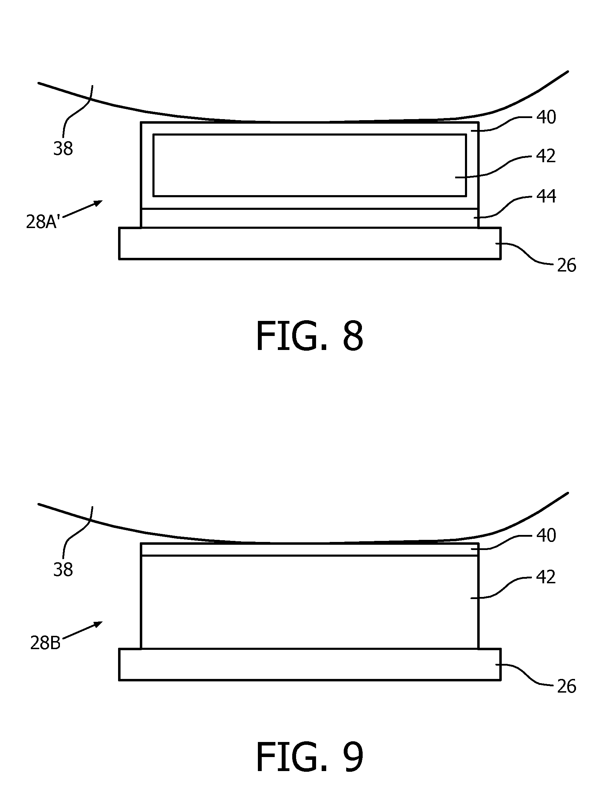

FIG. 8 is a schematic representation of forehead cushion 28A' according to an alternative particular, non-limiting exemplary embodiment. In this embodiment, first layer 40, made according to any of Embodiments 40A-40F, encapsulates second layer 42, made according to either Embodiment 42A or 42B.

FIG. 9 is a schematic representation of forehead cushion 28B according to an alternative particular, non-limiting exemplary embodiment. In FIG. 9, forehead cushion 28B is shown as engaging the forehead 38 of the patient. In addition, as seen in FIG. 9, forehead cushion 28B according to the present embodiment has a two layer rather than a three layer configuration comprising a first layer 40 structured to engage the skin, and a second layer 42 structured to provide a load distribution and/or an air or water transport functionality (third layer 44 has been omitted in this embodiment). Any of Embodiments 40A-40 F can be used to implement first layer 40 and any of Embodiments 42A, 42B can be used to implement second layer 42. In this embodiment, second layer 42 is directly attached to forehead support bracket 26.

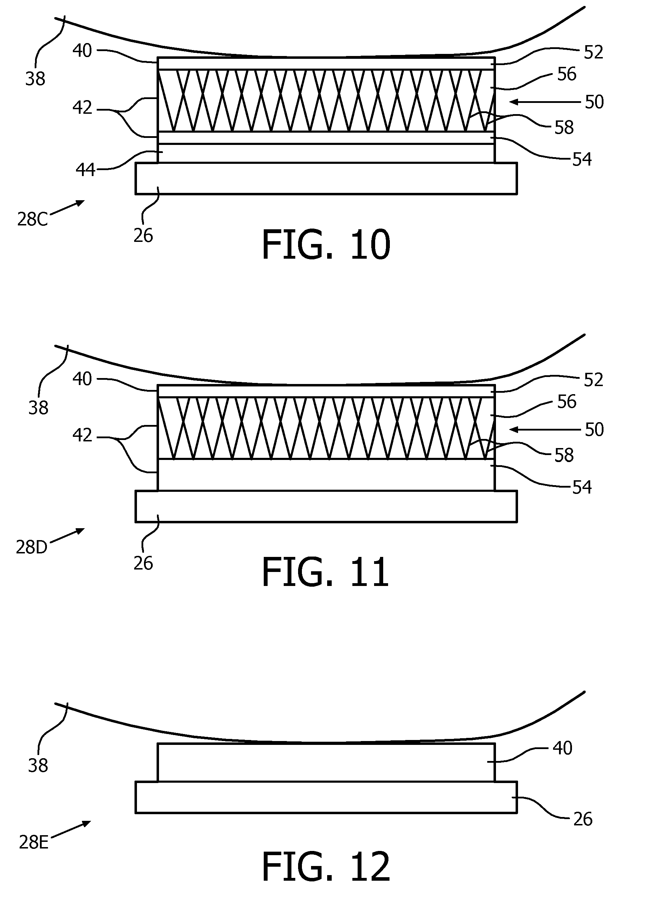

FIGS. 10 and 11 are schematic diagrams of further alternative embodiments of forehead cushion 28, labeled 28C and 28D, respectively. In forehead cushions 28C and 28D, as seen in FIGS. 10 and 11, a spacer fabric structure 50 is used to together implement first layer 40 and second layer 42. More specifically, upper fabric layer 52 is use to implement first layer 40 (the skin contacting layer), and inner layer 56 (comprising fibers 58) and lower fabric layer 54 together form second layer 42 (the load distribution layer). In such an implementation, any of the textile embodiments of first layer 40 described herein (Embodiments 40A-40F) may be used to implement upper fabric layer 52.

FIG. 12 is a schematic diagram of yet a further alternative embodiment of forehead cushion 28, labeled 28E. Forehead cushion 28E according to the present embodiment has a single layer rather than a two or three layer configuration comprising a textile layer 40 structured to both engage the skin and provide a low friction and a water management functionality and/or a load distribution functionality (second layer 42 and third layer 44 have been omitted in this embodiment). In this embodiment, layer 40 has a skin contacting surface having a coefficient of friction value of less than or equal to 1.0 (or 0.5) between the skin contacting surface and the skin. In addition, layer 40 may also have a maximum local stiffness of 100 kPa/mm, which corresponds to a minimum local compressibility of 0.01 mm/kPa, responsive to a maximum stress increase of $\leq$ 10 kPa ( $\approx$ redness threshold level) at an irregularity height d > 0.1 mm. Alternatively, layer 40 may also have the local stiffness properties set forth in TABLE 1 elsewhere herein. Any of materials of Embodiments 40A-40 F can be used to implement layer 40 as long as they satisfy either or both of the above described parameters. In this embodiment, as seen in FIG. 12, layer 40 is directly attached to forehead support bracket 26.In addition, the concepts described in connection with FIGS. 3-12 are not limited to use with just forehead pads. Rather, such concepts may also be used to implement other types of cushions that may be used in a patient interface device, such as, without limitation, cheek cushions/pads and chin cushions/pads (including flaps that may form a part thereof). Like a forehead cushion, those types of cushions typically do not have air flowing through them, and thus there is no need to ensure a near air tight seal between the cushion and the skin. In addition, in some applications, the concepts described in connection with FIGS. 3-12 may even be able to be used unmodified with a sealing cushion for a facial mask (including flaps that may form a part thereof). Such an application, however, may not provide optimum sealing functionality. Thus, a particular alternative implementation for such a mask that provides enhanced sealing capability is described below.

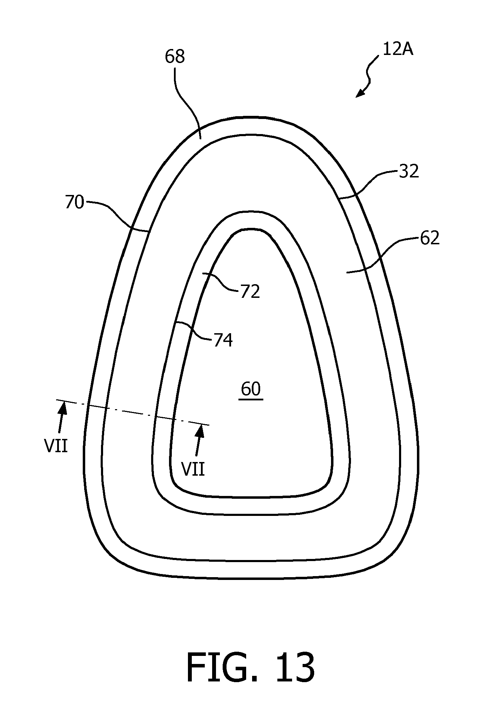

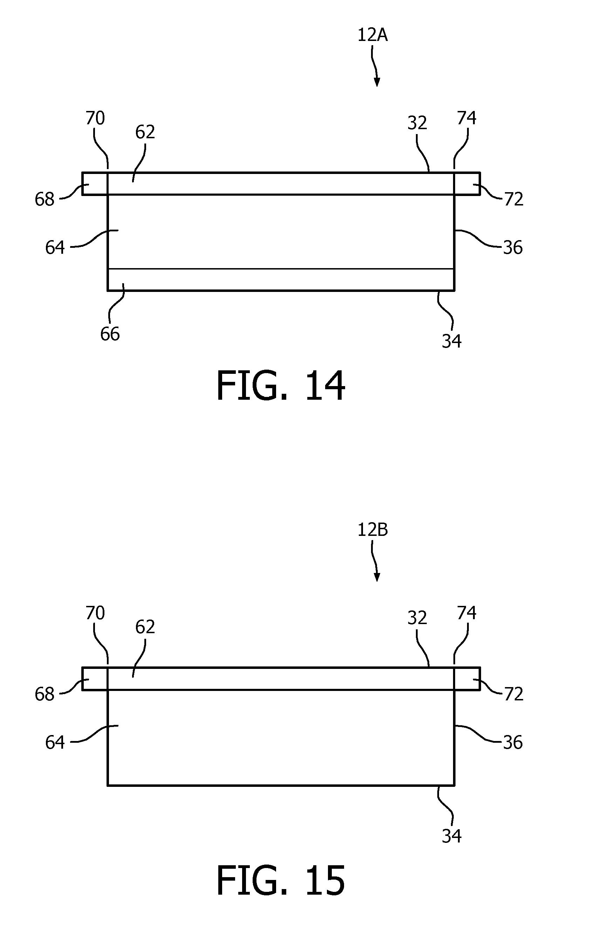

FIG. 13 is a front elevational view and FIG. 14 is a cross sectional view (taken along lines VII-VII if FIG. 13) of cushion 12 (labeled 12A in FIGS. 13 and 14) that is attached to frame assembly 10 (FIGS. 1 and 2) according to one particular exemplary embodiment of the present invention. In this embodiment, the concepts described elsewhere herein with respect to FIGS. 3-12 have been adapted in order to be especially suitable for use in a facial sealing cushion application (including flaps that may form a part thereof). As noted elsewhere herein, in the illustrated embodiment, respiratory mask 4 is a nasal/oral mask structured to cover the nose and mouth of the patient, and thus cushion 12A is structured to cover the nose bridge, some areas on the cheek and the chin of the user. For cushion 12A, there is a need to fulfil the functionality requirements of a PAP mask, i.e., management of required air flow, prevention of excessive air leakage and providing stability of the mask on the user's face/head during therapy. This will have consequences for the design and material choice for cushion 12A. Contrary to forehead pad 28 (e.g., in embodiments 28A-28D) as described herein, there is therefore a need to ensure the (near) air-tight connection between cushion 12A and the skin of the user.

Generally, all of the designs and concepts described in connection with forehead pad 28 (e.g., in embodiments 28A-28D) are suitable for cushion 12A as well. However, it is advantageous in the exemplary embodiment to introduce some means of partial or complete sealing into the design in order to achieve the functionality requirements of respiratory mask 4 combined with comfort requirements as described in the background section hereof. Thus, as described in detail herein, cushion 12A in the exemplary embodiment is provided with a means of sealing in order to make the designs and concepts described in connection with forehead pad 28 (e.g., in embodiments 28A-28D) more suitable for facial sealing cushion application. The main function of the sealing mechanisms is to make the cushion 12A nearly air tight, meaning that a maximum of 60-70%, and preferably 90%, of the vent flow takes the required pathway and a maximum of 30-40%, but preferably only up to 10%, of the flow is lost through the textile interface around the entire outer and/or inner edges of the facial pad or selective areas on the outer and/or inner edges.

As noted elsewhere herein (in connection with FIG. 2), cushion 12A includes a first end portion 32 structured to sealingly engage the patient's face, a second end portion 34 opposite first end portion 32 that couples to the rear of frame assembly 10, and a sidewall portion 36 extending between first end portion 32 and second end portion 34 such that cushion 12A defines an inner chamber, labeled as 60 in FIG. 13. In addition, cushion 12A in the illustrated embodiment, like forehead cushion 28A, has a three layer configuration comprising a first layer 62 structured to engage the skin, a second, intermediate layer 64 structured to provide a load distribution and/or an air or water transport functionality, and a third layer 66 structured to engage and facilitate attachment to faceplate portion 11 of frame assembly 10. First layer 62 may be made using any of the materials and/or configurations of Embodiments 40A-40F described in greater detail elsewhere herein. Second layer 64 may be made using any of the materials and/or configurations of Embodiments 42A-42B described in greater detail elsewhere herein. Finally, third layer 66 may be made using any of the materials and/or configurations described in detail herein in connection with third layer 44.

Furthermore, as seen in FIGS. 13 and 14, cushion 12A includes a first sealing element 68 coupled to the outer edge 70 of cushion 12A (to first layer 62 as shown or alternatively to second layer 64) such that it extends around (partially or completely) the outer perimeter of cushion 12A. Cushion 12A in the illustrated embodiment also includes a second sealing element 72 coupled to the inner edge 74 (that defines chamber 60) of cushion 12A (to first layer 62 as shown or alternatively to second layer 64) such that it extends around (partially or completely) the inner perimeter of cushion 12A. First sealing element 68 and second sealing element 72 ensure that cushion 12A provides a sufficient, near air tight seal during use of respiratory mask 4. In one particular embodiment, the sealing element(s) 68 and/or 72 are structured to permit no more than 40% (and in one implementation no more than 10%) of the vent flow to leak from cushion 12. Sealing elements 68 and 72 may be made from a number of different materials and/or have a number of different structures and configurations. A number of exemplary embodiments of first sealing element 68 and second sealing element 72 are described below.

In one exemplary embodiment, first sealing element 68 and/or second sealing element 72 may be formed by printing or coating edge 72 or 74, as the case may be, with a narrow band of silicone or another polymeric material such as, without limitation, polyurethanes, adhesives, or wax. In another exemplary embodiment, first sealing element 68 and/or second sealing element 72 may comprise a band of self-adhesive material (e.g., medical grade) coupled to edge 72 or 74, as the case may be, in order to achieve a skin tight contact around edge 72 or 74. In one exemplary implementation, the self-adhesive layer can be added externally, and would thus be a removable and disposable layer.

In yet another exemplary embodiment, first sealing element 68 and/or second sealing element 72 comprise an extra band of a textile material (e.g., a fabric) or a complete textile (e.g., fabric) layer at edge 72 or 74, as the case may be, that is bonded with first layer 62 or second layer 64 in the areas that are required to provide essential functionality such as preventing excessive leakage (for example under the eyes or around the mouth). Bonding of the extra textile band/layer of this embodiment can be achieved by various methods, such as, without limitation, thermal bonding (lamination), bonding during fabric formation (double/multiple knit/weave that are bonded by internal stitching (e.g. in a double jersey knit or multiple layer weave)), and stitching and/or embroidery in single or multiple rows and desired patterns.

In still another exemplary embodiment, first sealing element 68 and/or second sealing element 72 can be a tubular shaped edge member formed out of, for example, foam, gel, or a woven, knitted or nonwoven textile material. On one exemplary implementation, the first sealing element 68 and/or second sealing element 72 will have an openness of 0-60% or 10-40%. Also, the first sealing element 68 and/or second sealing element 72 may be coated or laminated with a polymer material. If the tubular shaped edge member of the present embodiment is made from a textile material, it can be knitted with the other parts of cushion 12A as one piece, for example as part of first layer 62. Alternatively, if tubular shaped edge member of the present embodiment is made out of, for example, foam or gel, it could be attached externally to first layer 62 or produced as a seamless part of second layer 64. Finally, if second layer 64 is a spacer textile material (i.e., spacer fabric) as described elsewhere herein, the tubular shaped edge member can be provided as part of the spacer textile material, and may have an openness of 0-60% or 10-40%, and or be coated or laminated with a polymer material.

In another exemplary embodiment, first sealing element 68 and/or second sealing element 72 can be an edge member formed by creating a density gradient (selvedge effect) in first layer 62 (made of textile) and/or the spacer textile embodiment of second layer 64 wherein the edge portion forming first sealing element 68 and/or second sealing element 72 is made of the same material yet has a higher density than the rest of first layer 62 or second layer 64, as the case may be. In particular, in this embodiment, the density of first sealing element 68 and/or second sealing element 72 is chosen to be high enough to prevent excessive air leakage from respiratory mask 4 (e.g., such that a maximum of 60-70%, and preferably 90%, of the vent flow takes the required pathway and a maximum of 30-40%, but preferably only up to 10%, of the flow is lost through the textile interface). Alternatively, an openness gradient could also be applied in second layer 64 made of foam as described elsewhere herein.

FIG. 15 is a cross-sectional view (similar to the cross-sectional view of FIG. 14) of cushion 12 (labeled 12B in FIG. 15) that may attached to frame assembly 10 (FIGS. 1 and 2) according to another, alternative particular exemplary embodiment of the present invention. As seen in FIG. 15, cushion 12B according to the present embodiment has a two layer rather than a three layer configuration comprising a first layer 62 structured to engage the skin, and a second layer 64 structured to provide a load distribution and/or an air or water transport functionality as described in greater detail herein (third layer 66 has been omitted in this embodiment). In this embodiment, second layer 64 is directly attached to faceplate portion 11 of frame assembly 10. In addition, in this embodiment, first sealing element 68 and/or second sealing element 72 can be according to any of the embodiments thereof that are described herein.

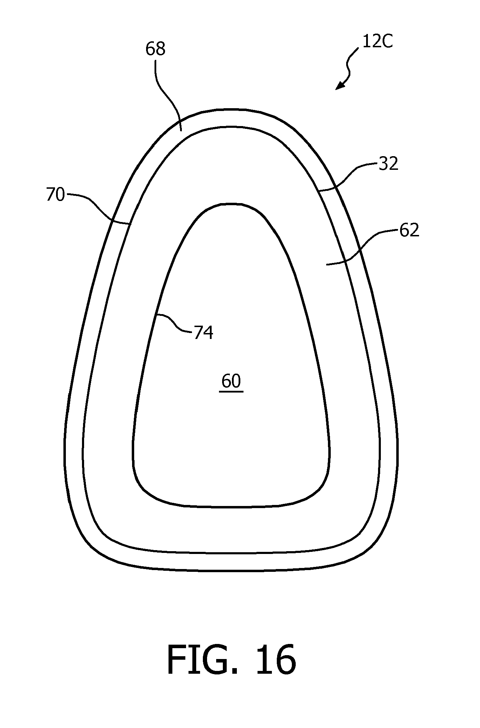

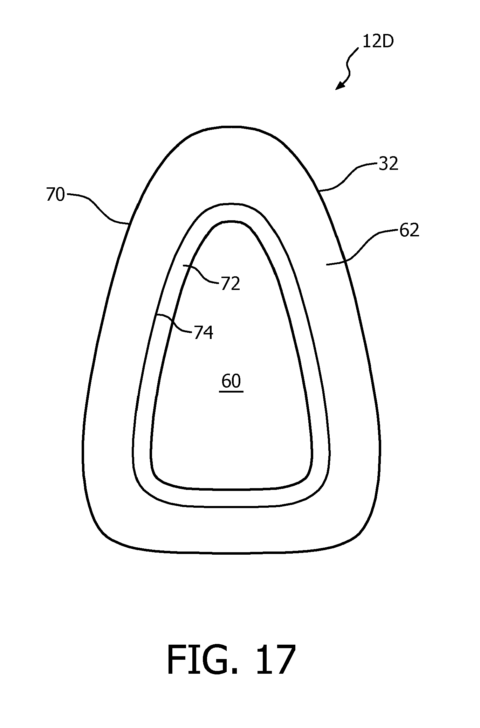

FIG. 16 is a front elevational view of a cushion 12C that may be attached to frame assembly 10 (FIGS. 1 and 2) according to an alternative particular exemplary embodiment of the present invention. Cushion 12C is similar in structure to either cushion 12A or 12B described above, except that cushion 12C only includes first sealing element 68 (and not second sealing element 72). FIG. 17 is a front elevational view of a cushion 12D that may be attached to frame assembly 10 (FIGS. 1 and 2) according to still a further alternative particular exemplary embodiment of the present invention. Cushion 12D is similar in structure to either cushion 12A or 12B described above, except that cushion 12D only includes second sealing element 72 (and not first sealing element 68).

In still another non-limiting exemplary embodiment, a patient interface design takes into account the fact that cushion 12 potentially covers several areas of the user's face that are not homogeneous in their properties. The nose bridge, for example, lacks the layer of fat that might be present on the cheek and function as a force distribution layer. At the same time, movement of cushion 12 might not affect all areas in the same manner. This might mean that certain areas are more prone to irritation caused by skin friction than other areas.

It is therefore proposed, in this embodiment, to have dedicated design parameters for different areas of the face covered by cushion. These design parameters are listed below.

In an apex region of cushion 12 that is structured to cover the nose bridge and be positioned below the eyes, a higher level of force distribution is provided by a portion of second layer 64 (spacer fabric, foam, nonwoven, gel) that has a higher thickness and/or compressibility than in the remainder (the bottom region and the first and second opposite side regions) of cushion 12. In addition, in this embodiment, the apex region may also have a different level of sealing as compared to the remainder of the cushion 12 in order to prevent blowing air in the eyes. Moreover, since skin structure under the eyes is different, resulting in more delicate skin, this embodiment may also provide a first layer 62 having high smoothness in the fabric to avoid skin friction and/or deformation as described herein (a coefficient of friction value of less than or equal to 1.0 (or 0.5) between the skin contacting surface and the skin of the patient in the apex region, wherein the reminder may have a coefficient of friction value of greater than 1.0 (or 0.5) between the skin contacting surface and the skin of the patient, for example as measured as described elsewhere herein).

Furthermore, if a head gear (strap) is used to fix the cushion 12 to the head of the patient, it would mean that there might be movement induced skin deformation in the areas covering the check and chin. In the exemplary embodiment, those areas correspond to the bottom region and the first and second opposite side regions of cushion 12. Thus, in the present embodiment, those areas may be provided with a first layer 62 having optimum surface smoothness (a coefficient of friction value of less than or equal to 1.0 (or 0.5) between the skin contacting surface and the skin of the patient). In this embodiment, the apex region may or may not also have this same optimum smoothness as discussed above.

Finally, sealing requirements in the cheek area are mostly driven by the mask functionality as moderate air flow on the skin can be perceived as not negative or even positive since it cools the skin down. In the chin area, however, sealing should inhibit air flow around the mouth in order to avoid dryness of the mouth that can disrupt sleep. Thus, in this embodiment, the bottom region of cushion 12 may have a different level of sealing in order to prevent blowing air around the mouth. In this embodiment, the apex region may or may not also have this same optimized sealing as discussed above.

The present application also relates to US application no. 61/502961, entitled "Skin Contact Product Having Moisture and Microclimate Control" (internal reference 2010PF01274) and to US application no. 61/586932 entitled "Medical and non-medical devices made from hydrophilic rubber materials" (internal reference 2011PF02736), both applications owned by the assignee of the present invention. The first application describes a user contacting assembly for use in, for example, a user interface device, that includes (a) a support material, and (b) a contact structure comprising moisture uptake means that is non-releasably combined with and supported by the support material, wherein the contact structure is adapted so that the moisture uptake means at least partially contacts a skin surface of a user responsive to the user interface being worn by such a user, wherein the support material provides mechanical and dynamical stability for the moisture uptake means, and wherein the moisture uptake means allows for uptake or diffusion of moisture from a skin surface of a user over which the patient contacting assembly is disposed. More particularly, in one embodiment, a material system is provided that includes defined layers of hydrophilic and hydrophobic materials. The materials in embodiments described therein are preferably hydrophilic and hydrophobic silicone materials. Alternative hydrophilic materials are, for example, hydrophilic polyurethanes, but also may be moisture uptaking textiles such as cotton, silk or polyester with defined structure or hydrophobic textiles with hydrophilic coating. Alternative hydrophobic materials are latex or polybutadien. The second application describes use of the abovementioned materials outside the field of patient interfaces.

As described in that application, hydrophilic polyurethanes are made by coupling the diisocyanate monomer or pre-polymer with hydrophilic monomers or pre-polymers. Examples of hydrophilic monomers or pre-polymers are glycerol, ethylene glycol derivatives, polyethylene glycol and other hydroxyl function containing poly-ol compounds. The hydrophilic properties can be even further increased by coupling this small chain hydrophilic polyurethane with other hydrophilic polymers which do not necessarily contains a hydroxyl group. Examples of these more general hydrophilic polymers are: polyvinylpyrrolidones (usually with a number average molecular weight from 20,000 to 400,000), poly(hydroxyethyl methacrylates), polyethylene glycols (usually with a number average molecular weight from 200 to 10,000), polyvinyl alcohols (usually with a number average molecular weight from 10,000 to 150,000), polyacrylamides, alkali metal poly(meth)acrylates (such as, but not limited to, sodium polyacrylate, potassium polyacrylate, sodium polymethacrylate, potassium polymethacrylate), and mixtures thereof.

As noted above, the hydrophilic material may be a textile based material, in which the fiber content is inherently moisture absorbing such as cellulose fibers (cotton, viscose) or silk and wool. Alternatively moisture absorption can be achieved due to the textile structure such as woven, knitted, non-woven, or other engineered fabrics such as spacer fabric. In such an embodiment, the patient interface is either made fully out of the textile based material to take moisture up and reduce red marks or it can be a hybrid material with for example based on a rubber such as a silicone rubber combined with one or more layers of textile.

In one particular implementation, shown in FIG. 1 of that application, a material system is provided (that may be used to implement a user interface) that includes a hydrophobic material base layer and a hydrophilic material top layer. The hydrophilic material top layer is in contact with a moist surface, such as the user's skin. The hydrophilic material top layer comprises or consists of intrinsically hydrophilic material. The stiffness of hydrophilic materials can depend strongly on the water content. Typically, hydrophilic materials exhibit lower stiffness at higher water content. High water content occurs for hydrophilic materials which show a good water permeability. Therefore, the hydrophilic material top layer may be combined with the hydrophobic material base layer by placing the hydrophilic material top layer on top of the hydrophobic material base layer. Thus, the hydrophilic material top layer may allow for penetration or uptake of moisture from the skin by utilizing the hydrophilic nature of the polymers molecular framework or by allowing for passage of moisture through the material through dedicated channels. Accordingly, moisture accumulation in the skin may be prevented. For example, FIG. 6 of that related application shows that a reduction of moisture accumulation as compared to hydrophobic silicones may be obtained by using textiles such as silk or cotton.

In another particular implementation, shown in FIG. 2 of that application, a material system is provided (that may be used to implement a user interface) that includes a two phase compound of hydrophilic and hydrophobic materials. The material system includes a hydrophobic base material and hydrophilic material mixed into the hydrophobic base material. The outside of the material system may be formed of the hydrophobic base material, which may be perforated to include apertures. The apertures may connect the hydrophilic material with a moist surface, such as the user's skin. The material system in this implementation may by a composite mixture, where at least one hydrophilic material is combined with at least one hydrophobic base material. The hydrophilic material may allow for uptake and/or diffusion of moisture away from the interface of the material system with the skin. The hydrophobic base material may also provide the mechanical and dynamical stability of the material system.

In still another particular implementation, shown in FIG. 3 of that application, a material system is provided (that may be used to implement a user interface) that includes a material system with a horizontally stacked configuration of hydrophobic and hydrophilic materials. The material system in this implementation includes a hydrophobic base material that includes a plurality of holes positioned at an interface of the hydrophobic base material with a moist surface, such as the user's skin, and a hydrophilic material filling the holes. The hydrophilic material may come in contact with the skin and, thus, in contact with moisture. The hydrophilic material may accordingly allow for uptake and/or diffusion of moisture away from the contact area of the material system with the skin. The hydrophobic base material may provide the mechanical and dynamical stability of the material system.

In the embodiments in that application, as noted above, the hydrophilic material can be a textile integrated in the contact structure. Preferably such a material is adapted so that the textile at the skin surface of a user is crease-free and/or leak-free. The hydrophilic material can be a rubber material that takes up at least 5% by weight of water, preferably more that 10% by weight of water and particularly preferably more than 40% up to 120 % by weight of water, or up to 200% or up to 250% or up to 500% by weight of water after immersion in demineralized water at room temperature for a sufficient time such as 5 days or more to reach saturation. It is expected that with increasing water absorption the mechanical properties may be reduced such that a support material is not only necessary but must be designed in a form that will stabilize the hydrophilic material.

Moreover, the concepts of the various embodiment of the present invention described herein may be used in connection with the material systems of the above described related application. For example, the textile materials described herein (in connection with first layers 40 and 62) having the various particular parameters described herein may be used as the hydrophilic material in the material systems of the above described related application. Thus, in one particular, non-limiting example, a textile material made from a material having a skin contacting surface having a coefficient of static friction value of less than or equal to 2.0 between the skin contacting surface and the skin of the patient may be used as the hydrophilic material in the material systems of the above described related application. In another particular, non-limiting example, the various load distribution embodiments described herein (e.g., second layers 42 and 64) may be used to implement a part of the material systems of the above described related application to a provide a load distribution functionality responsive to the system being donned by the patient, wherein at least a portion of the material system will have a local stiffness of less than or equal to 100 kPa/mm responsive to a maximum stress increase on the cushion member of 10 kPa (or any of the properties set forth in TABLE 1 elsewhere herein).

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the spirit and scope of the appended claims. For example, it is to be understood that the present invention contemplates that, to the extent possible, one or more features of any embodiment can be combined with one or more features of any other embodiment.

**Claims**

1. A cushion member (12, 28) for a user interface device (4), comprising:

   at least a first layer (40, 62) structured to directly engage a skin surface of a user, the first layer comprising a textile material, wherein the first layer is capable of providing moisture uptake from human skin at a rate of at least 11 $g/m^2$.hr, wherein the textile material is at least one of (i) a hydrophilic material or a material rendered at least partially hydrophilic, wherein the hydrophilic material or the material rendered at least partially hydrophilic has a predetermined fabric density or a predetermined fabric weight that enables it to provide the moisture uptake from human skin at the rate of at least 11 $g/m^2$.hr, (ii) a hydrophobic material having a first predetermined openness that enables it to provide the moisture uptake from human skin at the rate of at least 11 $g/m^2$.hr, or (iii) a nonwoven fabric material made of hydrophilic fibers or mixed component fibers wherein at least one fiber is a hydrophilic fiber, wherein the nonwoven fabric material has a second predetermined openness that enables it to provide the moisture uptake from human skin at the rate of at least 11 $g/m^2$.hr.

2. The cushion member according to claim 1, wherein under (i) the predetermined fabric density is greater than or equal to 50 $g/m^2$ or the predetermined fabric weight is greater than or equal to 30 $g/m^2$ in order to provide the moisture from human skin at the rate of at least 11 $g/m^2$.hr, wherein under (ii) the first predetermined openness is 90-99%, wherein under (iii) the second predetermined openness is 75-90%.

3. The cushion member according to claim 1, wherein the cushion member is able to retain at least 88 $g/m^2$ of moisture.

4. The cushion member according to claim 1, wherein the textile material has a % moisture regain value of: (i) 7.0%-11.0% at 65% RH and 21°C temperature, or (ii) 11%-18% at 65% RH and 21°C temperature, and the material rendered at least partially hydrophilic has a % moisture regain value of 2-4% at 65% RH and 20°C temperature.

5. The cushion member according to claim 1, wherein the textile material is made from a material having a skin contacting surface having a coefficient of static friction value of one of: (i) less than or equal to 2.0 between the skin contacting surface and human facial skin, (ii) less than or equal to 1.0 between the skin contacting surface and human facial skin, or (iii) less than or equal to 0.5 between the skin contacting surface and human facial skin.

6. The cushion member according to claim 1, further comprising a second layer (42, 64) coupled to the first layer, the second layer being structured to provide a load distribution functionality responsive to the user interface device being donned by the user.

7. The cushion member according to claim 6, the second layer being selected from a group consisting of a nonwoven textile, a spacer fabric, a layer of foam and a layer of gel.

8. The user interface device according to claim 1, wherein the cushion member is structured such that a first moisture increase in and on human skin after 20 minutes of use of the cushion member wherein the cushion member engages the human skin will be in the order of 0.0 - 30.0 in arbitrary units, wherein the first moisture increase is determined by measuring skin hydration or skin moisture with a corneometer after the user interface device is applied to human skin for 20 minutes and subtracted from the skin hydration or skin moisture values measured with the corneometer before the user interface device is applied to human skin, and wherein a second moisture increase in and on human skin would be after 20 minutes of use of a silicone-based user interface device employing a hydrophobic silicone cushion wherein the hydrophobic silicone engages the human skin in the order of 40.0 - 50.0 or more in arbitrary units, wherein the second moisture increase would be determined by measuring skin hydration or skin moisture with the corneometer after the silicone-based user interface device is applied to human skin for 20 minutes and subtracted from the skin hydration or skin moisture values measured with the corneometer before the silicone-based user interface device is applied to human skin.

9. The cushion member according to claim 1, wherein at least a portion of the cushion member has a local stiffness of less than or equal to 100 kPa/mm responsive to a stress increase on the cushion member of 1 kPa - 15 kPa.

10. The cushion member according to claim 6, wherein the second layer has a local stiffness responsive to a stress increase on the second layer of 1 kPa - 15 kPa of one of: (i) 100 kPa/mm or less, or (ii) 20 kPa/mm or less, or (iii) 7 kPa/mm or less, or (iv) 1 kPa/mm or less, or (v) 0.1 kPa/mm or less.

11. The cushion member according to claim 6, wherein the second layer has a first side and a second side opposite the first side, wherein the first layer is coupled to the first side, wherein the cushion member further comprises a third layer (44, 66) coupled to the second side, and wherein the third layer is a polymer layer structured to facilitate connection of the cushion member to a frame member of the user interface device.

12. The cushion member according to claim 9, wherein the cushion member comprises a fabric spacer having an upper fabric layer, a lower fabric layer, and an inner layer of fibers provided between the upper fabric layer and the lower fabric layer, wherein the first layer is the upper fabric layer and the second layer is the lower fabric layer and the inner layer of fibers.

13. The cushion member according to claim 9, wherein the cushion member is a sealing cushion (12), wherein the cushion member includes a first portion comprising the first layer and the second layer, the first portion having an inner edge (74) and an outer edge (70), the inner edge defining a chamber (60) of the sealing cushion, the cushion member further comprising a sealing element (68, 72) coupled to and extending from at least one of the inner edge and the outer edge of the first portion, wherein the sealing element is coupled to and extends from either or both of the first layer or the second layer,
wherein the sealing element is selected from a group consisting of: (i) a band of polymeric material, (ii) a band of self-adhesive material, (iii) a tubular band of foam, gel, woven textile or nonwoven textile material; (iv) an extension portion of the first layer or the second layer, wherein a density gradient is formed in either the first layer or the second layer such that extension portion has a higher density than a remaining a port of the first layer or the second layer from which it extends.

14. The cushion member according to claim 6, wherein the second layer exhibits a stress-displacement curve having a plateau region below a stress region of 0.6 to 1.0 N.cm$^{-2}$.

15. A user interface device (4) structured to deliver a flow of breathing gas to a user employing a cushion member according to any of the preceding claims.

FIG. 1

FIG. 2

FIG. 3

FIG. 4

Forehead pad

d

Spacer

$D_0$

Skin

F

d

D

$P_P$    $P_B$    $P_P$

# FIG. 5

FIG. 6

FIG. 7

FIG. 7A

FIG. 8

FIG. 9

FIG. 10

FIG. 11

FIG. 12

FIG. 13

FIG. 14

FIG. 15

FIG. 16

FIG. 17

Moisture increase at skin with different textiles

FIG. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61502961 B

- US 61586932 B

**Non-patent literature cited in the description**

- **M. KWIATKOWSKA ; S.E. FRANKLIN ; C.P. HENDRIKS ; K. KWIATKOWSKI.** *Wear,* 2009, vol. 267, 1264-1273